# EUROPEAN PATENT SPECIFICATION

(11) **EP 0 923 292 B1**
(45) Date of publication and mention of the grant of the patent: **02.07.2003**
(21) Application number: 97937544.1
(22) Date of filing: 01.08.1997
(51) Int. Cl.: A01N 49/00

(54) **IMPROVED METHOD FOR SYSTEMIC ADMINISTRATION OF 2,4-DIENOIC ACID-TYPE INSECTICIDES TO TERRESTRIAL MAMMALS**
VERFAHREN ZUR SYSTEMISCHEN VERABREICHUNG VON INSEKTIZIDEN VOM 2,4-DIENSÄURE-TYP AN TERRESTRISCHE SÄUGETIERE
METHODE AMELIOREE POUR ADMINISTRER PAR VOIE GENERALE DES INSECTICIDES DE TYPE ACIDE 2,4-DIENOIQUE A DES MAMMIFERES TERRESTRES

(30) Priority: 02.08.1996 US 23216 P; 20.11.1996 US 753148
(43) Date of publication of application: 23.06.1999
(73) Proprietor: Novartis AG, 4056 Basel (CH); Novartis Pharma GmbH, 1230 Wien (AT)
(72) Inventor: RUDOLPH, Robin, Richard, Grand Prairie, TX 75051 (US)
(74) Representative: Grubb, Philip William
(86) International application number: EP9704201
(87) International publication number: WO98005211

(56) References cited:
- EP-A- 0 255 803
- US-A- 4 850 305
- CHAMBERLAIN, WILLIAM F. ET AL: "Absorption, excretion, and metabolism of methoprene by a guinea pig, a steer, and a cow" JOURNAL OF AGRICULTURAL AND FOOD CHEMISTRY, vol. 23, no. 4, 1975, WASHINGTON,US, pages 736-42, XP002047305
- CHEMICAL ABSTRACTS, vol. 106, no. 11, 16 March 1987 Columbus, Ohio, US; abstract no. 80316, EL-GAZZAR, L. M. ET AL: "Insect growth regulators: mode of action on the cat flea, Ctenocephalides felis (Siphonaptera: Pulicidae)" XP002046785 & J. MED. ENTOMOL. (1986), 23(6), 651-4 CODEN: JMENA6;ISSN: 0022-2585, 1986,

## Description

### 1. Field of the Invention

This invention lies in the field of systemically active substances for the control of ectoparasites and endoparasites.

### 2. Background of the Invention

Various organic compounds are known to be active as systemic insecticides for the control of fleas in terrestrial mammals. Of particular interest are compounds for oral administration to dogs and cats to control, prevent or eliminate infestation by *Ctenocephalides felis* and *Ctenocephalides canis* (cat and dog fleas, respectively). Included among these compounds are juvenile hormones and compounds chemically similar thereto, benzoylurea derivatives, and triazine derivatives. Included among the juvenile hormones are 2,4-dienoic acids and phenoxyphenoxy compounds, particularly phenoxyphenoxyalkoxy-heterocyclics. Examples of 2,4-dienoic acids and related compounds are methoprene, hydroprene, neotenin, and epiphenonane. Examples of phenoxyphenoxy compounds are fenoxycarb and pyriproxyfen. Examples of benzoylureas are lufenuron, diflubenzuron, terflubenzuron, triflumaron, hexaflumaron, and flucycloxuron. An example of a triazine derivative is 2-cyclopropylamino-4,6-bis(dimethylamino)-*s*-triazine.

These compounds and others related in structure and of similar activity were originally disclosed for use by direct application to fleas. Subsequent studies showed that activity could also be obtained by systemic application of the compounds to the host animal. These studies are disclosed in Bamett *et al*. (Ciba-Geigy Corporation), United States patent no. 4,973,589 (November 27, 1990); Barnett *et al*. (Ciba-Geigy Corporation), United States patent no. 5,416,102 (May 16,1995); and Miller (Virbac, Inc.), United States patent no. 5,439,924 (August 8, 1995).

According to these patents and the technical literature published by the suppliers of insecticides addressed by these patents, the prevention of adult flea development is achieved when the parental fleas feed on the blood of the host animal. Adult fleas feed directly on the blood through the epidermis of the animal, while flea larvae feed on the partially digested blood present in the feces of the adult fleas. Insecticidal activity is thus purportedly achieved by maintaining an insecticide concentration in the blood that exceeds what is perceived as a minimum effective concentration. Since the minimum effective concentrations have not been verified by means other than administering the insecticides to live animals and observing the effect on fleas and flea eggs in the animals' coats, it is assumed that the dosage to a host animal that results in ovicidal activity against flea larvae in the animal's immediate surroundings produces a lethal amount of the insecticide in the animal's blood.

### SUMMARY OF THE INVENTION

It has now been discovered that certain insecticides upon oral administration to the host animal continue to be effective against fleas long after the quantity of insecticide in the blood of the animal is no longer detectable. It has further been discovered that when fleas are fed directly (and only) on the blood taken from the host animal but out of contact with the animal's coat, the concentration that is required to achieve insecticidal or ovicidal activity is considerably greater than the concentration observed in the blood when the fleas are killed by systemic administration to the animal. Accordingly, for these insecticides, the present invention resides in the control of fleas and flea eggs by systemic administration to the host animal at dosages that are not sufficient to maintain what has previously been considered to be the minimum effective concentration in the blood, but are still sufficient to achieve the insecticidal or ovicidal effect. Thus, in contradiction to the prior art, effective flea control with these insecticides is achieved by systemic administration at levels considerably lower than those needed to exceed a minimum concentration in the host animal blood.

Details on these and other features and advantages of the invention will become apparent from the description that follows.

### DETAILED DESCRIPTION OF THE INVENTION AND PREFERRED EMBODIMENTS

This invention is applicable to the use of 2,4-dienoic acids, salts or esters of the formula 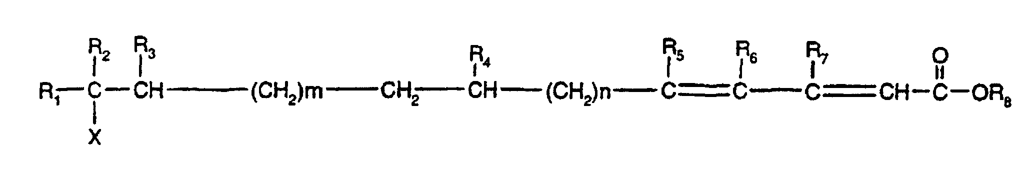 in which:
R¹ is C₁-C₆ alkyl;
R² is H, methyl or ethyl;
R³ is H or methyl;
R⁴ is methyl or ethyl;
R⁵ is H or methyl;
R⁶ is H or methyl;
R⁷ is methyl or ethyl;
R⁸ is a H, C₁-C₆ alkyl, C₃-C₆ alkenyl, C₃-C₆ alkynyl, C₃-C₈ cycloalkyl, phenyl, naphthyl, C₇-C₁₂ aralkyl, or cations of lithium, sodium, potassium, calcium, strontium, copper manganese, or zinc;
X is Br, Cl, Fl or OR⁹, in which R⁹ is H, C₁-C₆ alkyl, or C₁-C₆ alkanoyl;
m is zero, 1, 2, or 3; and
n is zero, 1,2, or 3.

As used herein, the term "cycloalkyl" refers to a saturated hydrocarbon ring, including rings with one or more alkyl groups branching off from a ring carbon. Examples are cyclopropyl, cyclopentyl, cyclohexyl, methylcyclohexyl, and cyclohexylmethyl. Preferred cycloalkyl groups are C₃-C₆ cycloalkyl, with cyclopentyl and cyclohexyl particularly preferred. The term "aralkyl" refers to an alkyl group substituted with an aromatic group both with and without one or more additional alkyl groups branching off from a ring carbon. Examples of aralkyl groups are benzyl, phenylethyl, naphthylmethyl, and ethylbenzyl. Preferred aralkyl groups are C₇-C₉ aralkyl, with benzyl and phenylethyl particularly preferred. The term "alkanoyl" refers to an alkyl group bonded to a carboxy group. Examples are acetyl, propionyl, butyryl, and hexanoyl. Preferred alkanoyl groups are C₁-C₃ alkanoyl, with acetyl and propionyl particularly preferred.

Within the scope of the above formula for 2,4-dienoic acids, salts and esters, certain subgenera are preferred. One such subgenus, for example, is defined as that in which the double bonds are in an *E,E* or *Z,E* configuration, and most preferably an *E,E* configuration. Another such subgenus is defined as: R¹ is methyl or ethyl, R² is methyl or ethyl, R⁷ is methyl, R⁸ is C₁-C₆ alkyl or C₃-C₆ alkynyl, X is chloro or OR⁹, m is zero or 1, and n is 1, all other variable groups as defined above. A third such subgenus is defined as: R¹ is methyl or ethyl, R² is methyl, R³ is H, R⁴ is methyl, R⁵ is H, R⁶ is H, R⁷ is methyl, R⁸ is C₁-C₄ alkyl or C₃-C₄ alkynyl, X is chloro or OR⁹, m is 1, and n is 1. A fourth preferred subgenus is the same as the third, except that R⁹ is H, methyl, ethyl, isopropyl, *t*-butyl, or acetyl. A fifth is also the same as the third, except that R⁹ is methyl, ethyl, isopropyl, or *t*-butyl. Specific examples of known compounds within the scope of the formula are 1-isopropyl (*E,E*)-11-methoxy-3,7,11-trimethyl dodecadi-2,4-enoate (methoprene, alternate name *trans*(2),*trans*(4)-isopropyl 11-methoxy-3,7,11-trimethyldodeca-2,4-dienoate), methyl (*E,E*)-3,7,11-trimethyl dodecadi-2,4-enoate (hydroprene), and 2-propynyl (E,E)-3,7,11-trimethyldodeca-2,4-dienoate (kinoprene). Methoprene and particularly (*S*)-methoprene are of particular interest. In the case of hydroprene, the preferred optical configurations are (*R,S*) and (*S*), while for kinoprene the preferred configuration is (*S*).

A goal of this invention is to achieve at least about 80% lethality of fleas that are in contact with the skin or hair of the animal, yet with an amount of active ingredient that is insufficient to maintain a concentration in the blood of the animal that is about 50% lethal to fleas that emerge from adult fleas feeding directly on the blood. Within these parameters, the amount of active ingredient administered to the host animal as a single bolus or by daily dosage can vary considerably. In most applications, best results with the most efficient use of the compounds are achieved with a daily dosage of about 0.3 to about 10.0 milligrams active ingredient per kilogram of body weight of the animal (mg/kg) per day, or any dosage schedule resulting in approximately equivalent amounts in the animal's body. A preferred range is about 1.0 to about 5.0 mg/kg. Since measurement of the concentration in the animal's blood is generally an index of the amount present in the animal's body as a whole, preferred dosages can also be expressed in terms of amounts sufficient to maintain specified blood concentrations of the active ingredient. Expressed in these terms, best results are generally obtained by administering an amount sufficient to maintain a lethal concentration of active ingredient in the hair or fat tissue of the animal but not sufficient to maintain a concentration of 70 parts per billion on a weight basis in the blood.

While not intending to be bound by theory, it is believed that the active ingredient is partitioned between the blood and certain tissues and glands in the animal's body, with preferential affinity for these tissues and glands, particularly lipophilic tissue such as fat cells. It is possible that a significant, and even a major, portion of the efficacy is achieved by passage of the active ingredient to the flea or flea egg through the lipophilic tissue, sebaceous glands and aprocrine glands, rather than the blood or hydrophobic tissue.

The active ingredient can be formulated in any conventional manner for administration to the host animal. The choice of formulation will depend on the type of animal, the size of the animal, and the method of administration, among other factors such as convenience and cost. Examples of different formulations are powders, tablets, granules, capsules, and emulsions. For oral administration, the active ingredient can be combined with the animal's feed by simple mixing, or by incorporation into or enrobement of pellets, chunks or particles of food matter. Examples are biscuits, treats, or chewable tablets enrobed or impregnated with the active ingredient, and liquid forms of the active ingredient that can be dispersed in water. The active ingredient can further be supplemented with adjuvants conventionally employed as formulating aids, as well as those that stimulate voluntary ingestion by the animal, such as scents or flavorings. Examples of adjuvants for inclusion in the formulations are fillers and binders including sugars such as lactose, saccharose, mannitol or sorbitol, cellulose preparations, calcium phosphates, starches such as corn, wheat, rice, or potato starch, gelatin, tragacanth, methylcellulose, agar, and sodium alginate. Other examples are flow-regulating agents and lubricants, such as silica, talc, stearic acid and salts thereof, and polyethylene glycol. Further examples will be readily apparent to those skilled in the formulations art.

Administration can be also achieved parentally or by implant. Parenteral administration can be achieved by subcutaneous, intravenous, or intramuscular injection, or by transdermal application. Implants are prepared for example by dispersing the active ingredient through a matrix such as solid rubber from which the active ingredient can leach out or placing it in a hollow capsule with walls through which the active ingredient can diffuse. All such formulations and methods of administration are known among those skilled in the art.

This invention is application to the treatment of terrestrial mammals, the scope and meaning of which is well known among those skilled in the art of animal husbandry and pets. Included among this class are household pets, livestock, and various companion animals. Specific examples, are dogs, cats, hamsters, and ferrets. Of particular interest are dogs and cats, with perhaps the greatest interest being dogs.

The methods of this invention are applicable to a use for the elimination or reduction of a flea infestation, by administration to an animal suffering from such an infestation. The methods are also applicable to the prevention of a flea infestation, by administration to an animal that is not so infested but positioned in or expected to enter an environment in which the dog or cat would otherwise be susceptible to such infestation. The term "controlling fleas" is used herein to denote both elimination or reduction of an infestation already present and prevention of an infestation before it occurs.

The following examples are offered for purposes of illustration only.

### EXAMPLE 1

The following experiment demonstrates that (S)-methoprene when administered to dogs as a single oral dose does not remain in the dogs' blood for more than 48-96 hours. The dose administered in this experiment is the same dose shown in Example 2 below to be effective in controlling fleas for 14 days, again following a single oral dose.

Six healthy mixed breed dogs, three male and three female, of weight ranging from 14 to 22 kg per dog and ages ranging from 2 to 5 years, were utilized. For each dog, a gelatin capsule containing a measured volume of (S)-methoprene was prepared, based on the dog's weight to yield a dose of 50 mg/kg of the dog's body weight in a single gelatin capsule for each dog. The dogs were fasted 24 hours before administration of their individual capsules, and dosing was achieved by placing the capsule in the back of each dog's throat and causing the dog to swallow. Food was then withheld for an additional 8 hours, although water was always available, and the dogs were fed once the following morning.

At each sampling time, 50 mL of blood was collected from the femoral vein of each dog into two 25-mL syringes. Samples were taken seven days prior to dosing (to establish a baseline), followed by three hours, 1 day, 2 days, 4 days, and 7 days after dosing. Hematocrits were performed on each sample to assure that the hematocrit did not fall by more than 30% over the time period of the experiment.

To prepare the blood samples for methoprene content determinations, each 25-mL blood sample was mixed with 200 mL acetonitrile, 25 g anhydrous Na₂SO₄, and 10 g CELITE® in a blender for three one-minute segments, allowing the blender blades to cool for thirty seconds between segments. The mixture was then vacuum filtered, including a wash with 50 mL acetonitrile. The filtrate was extracted with petroleum ether for one minute, then diluted with 700 mL deionized water. To this was added 50 g NaCI and the solution was acidified to pH 2 with 0.5 N HCl. The aqueous phase was then discarded, and the petroleum ether phase was washed twice with 600 mL of deionized water. The petroleum ether was then filtered through a glass wool plug topped with 50 g Na₂SO₄. The Na₂SO₄ was then rinsed with 15 mL petroleum ether, and the extractant concentrated to 10 mL in a rotary evaporator, using a bath temperature of 30°C. The concentrated extractant was then stored in a refrigerator until the next step in the analysis.

Extraction columns were prepared by heat activating FLORISIL® (magnesium silicate absorbent) at 200°C for 24 hours, then allowing the FLORISIL to cool for 45 minutes, adding 5 mL of distilled water per 50 g of FLORISIL, shaking the mixture, and allowing it to equilibrate for 3 hours. A glass column with 28 mm intemal diameter was plugged with glass wool and filled with petroleum ether. A 1-cm layer of Na₂SO₄ was slowly poured into the column, followed by 6.5 cm of FLORISIL and 1.5 cm of Na₂SO₄. The petroleum ether was then drained until it reached the layer of Na₂SO₄. The extractant from the preceding paragraph was then loaded into the top of the column with a glass pipette, and its content rinsed with petroleum ether and added to the column. The column was then drained until the solvent layer was above the Na₂SO₄, and the eluant was discarded.
Elution was then performed with 1 L of 5% diethyl ether/petroleum ether for each sample, and fractions of 175 mL and 825 mL were collected. The 825 mL fraction was then evaporated to 5 mL and stored in a refrigerator.

To prepare the 825 mL fraction for gas chromatography, one mL of internal standard was added to the sample, and the sample volume was evaporated to approximately 0.3 mL with nitrogen. Two aliquots of 5 µL each were injected on a gas chromatograph (Perkin Elmer, Sigma 300, with hydrogen flame ionization detector and data system) equipped with a 6-foot-by 1/4-inch glass column with 3% OV-101 Chrom W HP 100/120 mesh packing, using nitrogen at 60 mUmin as the carrier gas, and injector, column and detector temperatures of 300°C, 166°C, and 280°C, respectively.

The results are shown in Table I below.

**TABLE I**

| Methoprene Blood Concentrations (µg/mL) in Dogs Following a Single Oral Dose of 50 mg/kg Body Weight | | | | | | | | | |
|---|---|---|---|---|---|---|---|---|---|
| Time (hours) | | Methoprene Concentration in Blood (mg/mL) | | | | | | Mean | SEM |
| | Dog: | A | B | C | D | E | F | | |
| | Sex: | M | F | F | F | M | M | | |
| 0 | | 0.000 | 0.000 | 0.000 | 0.000 | 0.000 | 0.000 | 0.000 | 0.000 |
| 3 | | 0.727 | 0.172 | nd | nd | 2.130 | 0.250 | 0.547 | 0.410 |
| 24 | | 0.263 | 0.096 | 0.151 | nd | nd | nd | 0.085 | 0.062 |
| 48 | | nd | nd | 0.346 | 0.197 | 0.074 | 0.006 | 0.104 | 0.071 |
| 96 | | nd | nd | * | nd | nd | nd | nd | nd |
| 168 | | nd | nd | nd | nd | nd | nd | nd | nd |
| "nd" : not detectable *: assay error | | | | | | | | | |

These results show that in two of the six dogs, the methoprene level fell below detectable amounts by 48 hours, and in the remaining four cases, the methoprene level fell below detectable amounts by 96 hours.

### EXAMPLE 2

This experiment demonstrates the oviddal efficacy of (S)-methoprene when orally administered to dogs as single doses of the same size as the dosage of Example 1 or less.

Nine random-bred, clinically healthy dogs, five male and four female, ranging in weight from 7.5 to 14.9 kg and in age from 7 months to 68 months, and having hair coats ranging from fine to coarse and from 1.0 to 2.5 cm in length, were used. The dogs were assigned to three treatment groups based on weight, one group designated to receive a placebo (gelatin capsules containing no (S)-methoprene), the second group to receive gelatin capsules individually prepared to contain the appropriate quantity of (S)-methoprene such that one capsule would provide 25.0 mg (S)-methoprene per kg of the body weight of a particular dog, and the third group to receive gelatin capsules individually prepared to provide 50.0 mg/kg of body weight per dog. Each dog was administered one capsule orally on Day 0 and a second capsule orally on Day 33, in addition to a regular feeding schedule of dog food and water.

Eleven days before the administration of the first gelatin capsule (*i.e*., on Day -11), and weekly thereafter through Day 59, each dog was infested with approximately 200 one-to two-week-old, unfed adult *C. fells* fleas of mixed sex ratio. Each dog was restrained for about one minute while the fleas were released along the dorsum.

Flea eggs were collected on Days -7, 0, 1, 2, 4, 7, and weekly thereafter through Day 63. Prior to egg collection, the cages in which the dogs were housed and the collection trays were rinsed with isopropyl alcohol, and the water was turned off, feed bowls and resting pads were removed from each cage. Trays covered with a screen made of 8-inch hardware cloth were placed under the cages, and heavy white butcher paper was placed under the hardware cloth. After an interval of 14-16 hours, the paper was swept and the debris and flea eggs thus collected were transferred to a metal pan. The debris was separated from the eggs by sieving, and the eggs were placed in half-pint plastic cartons, then transferred to 10 cm x 10 cm glass plates. Using a dissecting microscope and a fine tipped artist brush, 100 flea eggs were counted from the sweepings of each dog and separated into four replicates of 25 eggs each. The eggs were placed in a 15 mm x 60 mm disposable plastic petri dish and incubated at 79-80°F and 80% relative humidity. Seventy two hours after collection, larvae emergence was determined. Both dead and live larvae were counted to determine the ovicidal effect of the treatment.

The results are listed in Table II below, where each entry represents the percent inhibition of hatching of fleas from the flea eggs relative to the placebo group, and each entry is the average of all replicates and of all three dogs in the particular treatment group.

**TABLE II**

| Methoprene Systemic Flea Ovicidal Activity Upon Oral Administration to Host Dogs | | | |
|---|---|---|---|
| | | Percent Inhibition of Flea Egg Hatching | |
| Days After Initial Administration | Dosage (administered once on Day 0 and once on Day 33): | 50 mg/kg | 25 mg/kg |
| 1 | | 98.9 | 92.3 |
| 2 | | 100.0 | 80.4 |
| 4 | | 100.0 | 100.0 |
| 7 | | 97.5 | 44.4 |
| 14 | | 97.9 | 75.1 |
| 21 | | 73.0 | 79.0 |
| 28 | | 50.8 | 39.9 |
| 35 | | 100.0 | 100.0 |
| 42 | | 100.0 | 95.9 |
| 49 | | 51.5 | 11.3 |
| 55 | | 31.9 | 12.6 |
| 63 | | 16.1 | 14.0 |

These results show that (S)-methoprene is ovicidally effective on fleas when administered systemically to host dogs, at both the dosage used in Example 1 and at half that dosage, and that the ovicidal efficacy lasts for two weeks or more, well past the time by which the (S)-methoprene concentration in the blood of the dogs drops below detectable levels.

### EXAMPLE 3

This experiment compares the ovicidal efficacy of (S)-methoprene when orally administered to dogs with the ovicidal efficacy of (S)-methoprene in dog blood drawn from the same dogs and fed directly to fleas in an *in vitro* administration. The (S)-methoprene was administered in initial bolus dosages followed by daily administration at low rates.

For the *in vitro* tests, an artificial feeding system with cages for adult cat fleas *C. felis* was used as described by Wade, S.E., *et al*., *J. Med. Ent.* **25**(3): 186-190 (May 1988). Inside each cage were placed 100 one-week-old, unfed adult *C. fells.* Each cage included a container for treated blood separated from the fleas by a PARAFILM® membrane, and a compartment containing thin strips of paper to support loosely arranged clean dog hair and to provide a foothold for the fleas as they fed on the blood through the membrane.

Fifteen healthy male beagle dogs, one year of age, with weights ranging from 10.29 kg to 13.87 kg, were divided into three groups of five dogs each. One group was fed an initial bolus of 50 mg/kg of (S)-methoprene, followed by daily feeding a standard maintenance diet containing 0.02% (S)-methoprene. The second group was fed an initial bolus of 25 mg/kg of (S)-methoprene, followed by daily feeding a standard maintenance diet containing 0.01% (S)-methoprene. The third group received the same diet but with no (S)-methoprene, thereby serving as a control. The daily feeding of 0.02% (S)-methoprene in the first group amounted to approximately 3.6 mg/kg, while the daily feeding of 0.01% (S)-methoprene in the second group amounted to approximately 1.8 mg/kg. The (S)-methoprene was administered in a gelatin capsule, the control involved administration of a gelatin capsule containing vegetable oil instead of (S)-methoprene, and the standard diet was a Science Diet Canine Maintenance. The daily feeding of the (S)-methoprene continued for six weeks following the initial bolus.

Seven days after the initial bolus and weekly thereafter for five more weeks except week 4, 50 mL of blood was collected from each dog into a syringe preloaded with 3 mL of a 20% (by weight) aqueous sodium citrate solution. The five 50-mL samples from each treatment group were pooled at each bleeding. Aliquots of 10 mL each from each pool were placed in separate feeding chambers of the artificial flea feeding cages.

Flea eggs were collected from each cage at appropriate intervals. One hundred (100) healthy appearing fleas were counted under a dissecting microscope from each cage, divided into two subsamples, and placed in 60 mm x 15 mm plastic petri dishes. The samples were incubated at 78°F and 85% relative humidity for three days, then scored for larval hatch by visual observation through the dissecting microscope. The data from all replicates in each treatment group were combined to yield the mean percent egg hatch, and the percent inhibition of egg hatch was calculated relative to the control.

Three days prior to the week 4 data point, each dog was infested with fleas in the manner described in Example 2. At week 4, flea eggs were collected from the dogs themselves in the manner described in Example 2, and the percent inhibition of egg hatch for the fleas collected in this manner was determined as in Example 2.

The *in vitro* results using the artificial feeding chambers are shown in Table III, and the *in vivo* results are shown in Table IV.

**TABLE III**

| (S)-Methoprene Flea Ovicidal Activity *In Vitro* | | | | |
|---|---|---|---|---|
| Weeks Following Start of Test | | Percent Inhibition of Flea Egg Hatching | | |
| | Initial Bolus: | 50 mg/kg | 25 mg/kg | Control |
| 1 | | 33 | 6 | 0 |
| 2 | | --* | -- | -- |
| 3 | | 48 | 38 | 0 |
| 5 | | 38 | 24 | 0 |
| 6 | | 22 | 19 | 0 |

| | | | | |
|---|---|---|---|---|
| * insufficient flea egg production to determine hatch rates | | | | |

**TABLE IV**

| Methoprene Systemic Ovicidal Activity Upon Systemic Oral Administration to Host Dogs | | | | |
|---|---|---|---|---|
| Week Following Start of Test | | Percent Inhibition of Flea Egg Hatching | | |
| | Initial Bolus: | 50 mg/kg | 25 mg/kg | Control |
| 4 | | 100 | 100 | 0 |

These results collectively demonstrate that blood methoprene levels that are not sufficient to achieve effective ovicidal activity through the blood directly are the same blood levels that result from an ovicidally effective systemic administration. This proves that it is not necessary to maintain the concentration of methoprene in blood above a minimum ovicidal amount to achieve an ovicidal effect, and that the effect is achievable by systemic administration at dosages that result in blood levels below the ovidical amount.

### EXAMPLE 4

This experiment is a comparative experiment performed on an ovicide outside the scope of this invention. The ovicide is lufenuron (fluphenacur), and the tests performed in this experiment investigate the relationship between the ovicidal activity of this ovicide and its concentration in the blood of a mammal. This was achieved by causing fleas to feed directly on the blood containing the ovicide, in an *in vitro* artificial feeding system. The results of this experiment combined with those of Example 5 disprove the ovicidal mechanism asserted by the prior art.

The artificial feeding system and cages for adult cat fleas described in Example 3 were used. Beef blood was used for the feeding system, and was prepared by adding 35 mL of a 20% sodium citrate solution per liter of blood to prevent clotting. A stock solution of lufenuron was prepared by adding 50.1 mg of technical lufenuron to 4.951 g of dimethyl sulfoxide (DMSO) to yield a 1.0% solution. A 1,000 ppm solution was then prepared by diluting 1.0 mL of the 1.0% solution in 9.0 mL of DMSO, and a 100 ppm solution was prepared by diluting 1.0 mL of the 1,000 ppm solution in 9.0 mL of DMSO. Dimethyl sulfoxide itself was used as a control. To treat the blood, the various solutions (0.05 mL) were added to the citrated beef blood (100.0 mL) to yield beef blood solutions containing 5.0 ppm, 0.5 ppm, 0.05 ppm, and 0.00 (zero) ppm lufenuron. Each solution was mixed thoroughly, and 10 mL at each concentration were placed in each of five feeders in the artificial feeding system to serve as replicates. Each day fresh blood solutions identical to the originals were placed in the feeders.

Flea eggs were collected from each replicate on days 3, 5 and 7. One hundred (100) healthy appearing fleas were counted under a dissecting microscope from each replicate, divided into two subsamples per replicate, and placed in 60 mm x 15 mm plastic petri dishes. The samples were incubated at 78°F and 85% relative humidity for three days, then scored for larval hatch using by visual observation through the dissecting microscope. The data from all replicates at each concentration were combined to yield the mean percent egg hatch, and the percent inhibition of egg hatch was calculated relative to the control.

The results are shown in Table V below.

**TABLE V**

| Lufenuron Flea Ovicidal Activity *In Vitro* | | | | |
|---|---|---|---|---|
| Days Following Start of Test | | Percent Inhibition of Flea Egg Hatching | | |
| | Concentration of Lufenuron in Blood: | 5.0 ppm | 0.5 ppm | 0.05 ppm |
| 3 | | 100 | 80.6* | ** |
| 5 | | 99.8 | 56.5 | 10.8 |
| 7 | | 100 | 63.2 | 6.9 |

| | | | | |
|---|---|---|---|---|
| * Low number of eggs produced in both controls and treated groups | | | | |
| ** No eggs produced | | | | |

The 99 percentile effective concentration (EC99) for ovicidal activity of lufenuron lies between 0.5 ppm and 5.0 ppm when diluted in beef blood and fed to adult cat fleas directly (*in vitro*). This is to be compared with the results obtained in the next example.

### EXAMPLE 5

This is a further experiment performed on lufenuron to investigate the efficacy of lufenuron in oral systemic administration to host dogs.

Six random bred and purebred, clinically healthy dogs, three male and three female, ranging in weight from 5.5 kg to 17.2 kg each and in age from 5 months to 42 months, and having hair coats ranging from medium to coarse and from 1.0 to 5.0 cm in length, were used. The dogs were divided into two treatment groups of three dogs each. The dogs were all maintained on regular feeding schedules of dog food and water, and each dog in one of the two groups was administered an appropriate number of lufenuron-containing gelatin capsules to achieve a dosage of 10 mg/kg of the body weight of the dog. This dosage is identified in the supplier's technical literature as the minimum effective dosage for *C. felis* in dogs, and the dosage that will result in a blood concentration level of 0.05 ppm remaining for at least 14 days after the administration of a single dose. Administration was performed at Day 0 and again at Day 28.

On Days -8 (eight days before the lufenuron administration), -4, 3, 10, 17, 24, 31, 38, 45, 52, and 59, approximately 100 unfed adult *C. Felis* fleas were applied to each dog by standard procedures. Flea eggs were collected and larval emergence (egg hatching) was determined by the procedures of Example 2 on Days -4, 0, 1, 2, 4, 7, 14, 21, 28, 35, 42, 49, and 56. The results are listed in Table VI below.

**TABLE VI**

| Lufenuron Systemic Flea Ovicidal Activity Upon Oral Administration to Host Dogs Using Single Doses of 10 mg/kg at Day 0 and Day 28 | |
|---|---|
| Days Relative to Initial Administration | Percent Inhibition of Flea Egg Hatching |
| -4 | -- |
| 0 | -- |
| 1 | 39.9 |
| 2 | 70.6 |
| 4 | 54.5 |
| 7 | 54.1 |
| 14 | 52.1 |
| 21 | 10.7 |
| 28 | 0 |
| 35 | 48.6 |
| 42 | 0 |
| 49 | 72.7 |
| 56 | 20.5 |

Comparing these results with those in Table V above (Example 4), the percent inhibition of flea egg hatching upon oral systemic administration to host dogs is much higher than the percent inhibition upon direct *in vitro* feeding to the flea eggs at the same blood concentration. This in itself is a surprising result. Furthermore, since the technical literature shows that the concentration of lufenuron in blood does not drop below detectable levels within 48 hours of systemic administration as does that of methoprene, the continued efficacy of methoprene as shown in Examples 1 and 2 is even more surprising.

### EXAMPLE 6

Retuming to (S)-methoprene, this example presents *in vitro* flea ovicidal activity using beef blood, for comparison with Example 4. Procedures using beef blood were the same as those indicated in the previous examples. The results are shown in Table VII below.

**TABLE VII**

| (S)-Methoprene Flea Ovicidal Activity *In Vitro* (Using Beef Blood) | | | | | | |
|---|---|---|---|---|---|---|
| Days Following Start of Test | Concentration of (S)-Methoprene in Blood (ppm): | Percent Inhibition of Flea Egg Hatching | | | | |
| | | 0.5 ppm | 0.25 | 0.1 | 0.075 | 0.05 |
| 3 | | 99.1% | | | | 47.3 |
| 5 | | | 100.0 | 97.4 | | |
| 6 | | | 99.0 | 95.7 | | |
| 7 | | 100.0 | | | | |
| 8 | | | | | a: 66.0 | |
| | | | | | b: 78.0 | |
| 10 | | 100.0 | | | | 65.4 |
| 13 | | 100.0 | | | | |
| averages | | 99.8 | 99.5 | 96.6 | 72.0 | 56.4 |

### EXAMPLE 7

This example shows the distribution of (S)-methoprene between the blood and fat tissue of rats to which the (S)-methoprene was administered *in vivo.*

Sprague-Dawley CD® VAF/PLUS® male rats were purchased from Charles River Breeding Laboratories, Inc., Portage, Michigan, USA. The body weights of the rats averaged 200 g at the time of dose administration. The rats were quarantined for five days before use, except for jugular vein-cannulated rats, to which doses were administered 1 to 3 days after receipt. The rats were divided into three groups for dosage with ¹⁴C-(S)-methoprene:
Group A received a single dose of 10 mg/kg of the ovicide through intravenous injection via the jugular vein. Three rats each were sacrificed at 0.5 hour, 1 hour, 2 hours, 3 hours, 4 hours, 5 hours, 6 hours, and 7 hours after dosing.
Group B received a single dose 10 mg/kg of the ovicide through oral administration by gavage dosing. Three rats each were sacrificed at 1 hour, 2 hours, 3 hours, 4 hours, 5 hours, 6 hours, 7 hours, and 8 hours after dosing.
Group C received a single dose 100 mg/kg of the ovicide through oral administration by gavage dosing. Three rats each were sacrificed at 1 hour, 2 hours, 3 hours, 4 hours, 5 hours, 6 hours, 7 hours, and 8 hours after dosing.
   After dosing the rats were house individually in cages. Daily room temperature was maintained between 69°C and 75°C and the relative humidity was between 40% and 70%. All rats had free access to food and fresh tap water.

For analyses of (S)-methoprene in blood, about 7 mL of blood was extracted from each rat into 60 µL of heparin and 70 µL of paraoxon (to prevent decomposition of the (S)-methoprene). Each blood sample was mixed and hemolyzed with an ultrasonic sonicator for 1 minute, then extracted with 20 mL of a 1:1 (volume ratio) mixture of hexane and diethyl ether. The extract was sonicated for three minutes, vortex mixed for a further three minutes, then centrifuged. This procedure was repeated three times. The hexane/ether extracts were combined, radioassayed, evaporated on a rotary evaporator, and concentrated under a stream of nitrogen. Radioassays were performed by liquid scintillation counting, the samples having been decolorized prior to solvent extraction by mixing 100 µL of the blood with 20 µL hydrogen peroxide. Thin-layer chromatography (TLC) was performed on the concentrated blood samples, using precoated silica gel G chromatoplates 0.25 mm in thickness with fluorescence indicator. Organic extracts were spotted alone or cochromatographed with reference standards methoprene and methoprene acid, and then developed in hexane/diethyl ether/acetic acid 50:50:1 (volume ratios). Radioactive bands and spots were imaged and quantified.

For analyses of the ovicide in fat, visceral fat was collected from the sacrificed rats. The fat sample from each rat was thoroughly mixed 1:1 with cellulose powder, and homogenized with a few drops of water. A portion (0.5 to 1 g) of each fat sample was extracted with 10 mL of a mixture of hexane/diethyl ether (1:1), which was treated vigorously on a sonicator for four minutes, then vortex mixed for two minutes and centrifuged. This procedure was repeated twice. The extracts were then combined, radioassayed, rotary evaporated, and concentrated under nitrogen for TLC analysis. A small amount of methanol was added to precipitate the fat to improve the TLC separation. Radioassays and TLC analyses were performed in the same manner as for the blood samples.

Results for undecomposed (S)-methoprene (as isolated by TLC) are listed in Tables VIII and IX. A comparison of these tables indicates a striking imbalance in the distribution of the ovicide between blood and fat, with the larger proportion residing in the fat tissue.

**TABLE VIII**

| (S)-Methoprene Concentration in Rat Blood Upon *In Vivo* Administration | | | |
|---|---|---|---|
| Time (hours) | (S)-Methoprene Concentration in Blood (ppm)* | | |
| | Group A 10 mg/kg I.V. | Group B 10 mg/kg oral | Group C 100 mg/kg oral |
| 0.5 | 0.703 ± 0.307 | -- | -- |
| 1 | 0.345 ± 0.107 | 0.112 ± 0.038 | 0.165 ± 0.070 |
| 2 | 0.113 ± 0.028 | 0.264 ± 0.122 | 3.487 ± 1.324 |
| 3 | 0.079 ± 0.002 | 0.202 ± 0.065 | 1.302 ± 0.409 |
| 4 | 0.066 ± 0.028 | 0.130 ± 0.066 | 1.504 ± 0.533 |
| 5 | 0.048 ± 0.001 | 0.052 ± 0.026 | 0.930 ± 0.449 |
| 6 | 0.046 ± 0.027 | 0.045 ± 0.014 | 0.527 ± 0.341 |
| 7 | 0.019 ± 0.008 | 0.020 ± 0.005 | 0.608 ± 0.292 |
| 8 | -- | 0.021 ± 0.009 | 0.483 ± 0.221 |

| | | | |
|---|---|---|---|
| * Each entry in this table is an average of three animals. | | | |

**TABLE IX**

| (S)-Methoprene Concentration in Fat of Rat Upon *In Vivo* Administration | | | |
|---|---|---|---|
| Time (hours) | (S)-Methoprene Concentration in Fat (ppm)* | | |
| | Group A 10 mg/kg I.V. | Group B 10 mg/kg oral | Group C 100 mg/kg oral |
| 0.5 | 4.193 ± 1.566 | -- | -- |
| 1 | 6.936 ± 3.259 | 0.272 ± 0.103 | 1.060 ± 0.270 |
| 2 | 5.929 ± 1.197 | 1.138 ± 0.388 | 14.726 ± 3.371 |
| 3 | 5.233 ± 0.457 | 1.715 ± 0.680 | 22.868 ± 3.322 |
| 4 | 8.179 ± 3.532 | 2.327 ± 1.090 | 45.316 ± 16.910 |
| 5 | 7.596 ± 3.990 | 2.980 ± 0.657 | 38.420 ± 2.765 |
| 6 | 6.734 ± 2.209 | 3.369 ± 0.814 | 41.898 ± 11.568 |
| 7 | 8.077 ± 1.082 | 2.921 ± 1.345 | 39.393 ± 17.952 |
| 8 | -- | 2.408 ± 0.702 | 47.162 ± 20.116 |

| | | | |
|---|---|---|---|
| * Each entry in this table is an average of three animals with the exception of Group C at 1 hour, which is the average of two animals. | | | |

### EXAMPLE 8

This example compares the distribution of (S)-methoprene between the hair and the blood of dogs upon oral administration.

Twelve random-bred adult dogs of various sex and medium hair coat were selected based on hair coat, health, and individual habits. The twelve dogs were grouped into two groups of six dogs each. Dog weights ranged from 22.5 kg to 35.0 kg, and the various weights and each sex were represented in each group. The dogs received a measured amount of Science Diet maintenance dog food daily and water was available. The two groups of dogs were housed separately. The dogs of Group 1 were not given any treatments, whereas those of Group 2 were each given 50 mg/kg of technical (S)-methoprene one time only at the start of the study (Day 0).

Blood samples were taken periodically by drawing the samples into 10 cc disposable syringes and transferring them to labeled 5 mL heparinized EDTA vacutainers treated with 50 mL of paraoxon solution at a concentration of 1 x 10⁻² M. The blood samples were frozen until ready for analysis. Hair samples were clipped from the dogs on the same days that blood samples were taken, then placed in labeled freezer jars with aluminum foil seals, and frozen.

To analyze the dog hair, a known weight (4-8 g) of each dog hair sample was extracted in hexane, the extract evaporated and the residue reconstituted in 2 mL of hexane. One mL of the extract was cleaned by solid-phase extraction using a 3 cc/500 mg silica cartridge. The (S)-methoprene was then eluted from the cartridge with 5% ethyl acetate/hexane. The residue was reconstituted in 1 mL acetonitrile and analyzed by reversed-phase high-pressure liquid chromatography (HPLC) on a C₁₈ column (250 x 4.6 mm, 10-micron diameter) using diode array detection at a wavelength of 264 nm, with a mobile phase of 90:10 methanol:water at 1.5 mUmin and an injection volume of 100 mL. The (S)-methoprene peak eluted at about 5.1 minutes. The concentration of (S)-methoprene was calculated based on a calibration curve generated by concurrently running a series of standards. The limit of quantitation was about 20 ppb.

To analyze the blood samples, the samples were extracted three times with methyl *t*-butyl ether, evaporated, reconstituted in 1 mL of methanol, and analyzed by HPLC as in the hair analyses.

The results of the hair tests are shown in Tables X(A) and X(B), the latter presenting the control data. The results of the blood tests are shown in Tables XI(A) and XI(B), the latter presenting the control data. In each case, the results are reported in ppb, *i.e*., parts per billion by weight of the dog hair or of the whole blood. The letters "ND" indicate that the level of (S)-methoprene was below the limit of detection.

**TABLE X(A)**

| Concentration of (S)-Methoprene on Dog Hair Following Oral Administration at 50 mg/kg | | | | | | | |
|---|---|---|---|---|---|---|---|
| No. of Days | Dog No.: | (S)-Methoprene Concentration (ppb) | | | | | |
| | | #529 | #528 | #98 | #532 | #535 | #60 |
| 0 | | ND* | ND | ND | ND | ND | ND |
| 1 | | 86.6 | 76.6 | 47.4 | ND | 49.3 | 6000 |
| 3 | | 82.2 | 199 | 125 | 62.4 | 424 | 33,900 |
| 5 | | 58.3 | 114 | 161 | 98.6 | 276 | 12,500 |
| 7 | | 34.5 | 150 | 126 | 117 | 294 | 2,400 |
| 10 | | 60.5 | 126 | 167 | 255 | 681 | 12,300 |
| 14 | | 85.5 | 103 | 200 | 350 | 800 | 5,300 |
| 17 | | 39.7 | 137 | 345 | 331 | 1092 | 3,600 |
| 21 | | 93.5 | 31.2 | 231 | 342 | 675 | 12,100 |
| 28 | | 106 | 47.2 | 287 | 230 | 461 | 5,200 |

| | | | | | | | |
|---|---|---|---|---|---|---|---|
| * ND: below the lower limit of detection | | | | | | | |

**TABLE X(B)**

| Concentration of (S)-Methoprene on Dog Hair of Untreated Dogs | | | | | | | |
|---|---|---|---|---|---|---|---|
| No. of Days | Dog No.: | (S)-Methoprene Concentration (ppb) | | | | | |
| | | #380 | #527 | #531 | #534 | #536 | #538 |
| 0 | | ND | ND | ND | ND | ND | ND |
| 1 | | ND | ND | ND | ND | ND | ND |
| 3 | | ND | ND | ND | ND | ND | ND |
| 5 | | ND | ND | ND | ND | ND | ND |
| 7 | | ND | ND | ND | ND | ND | ND |
| 10 | | ND | ND | ND | ND | ND | ND |
| 14 | | ND | ND | ND | ND | ND | ND |
| 17 | | ND | ND | ND | ND | ND | ND |
| 21 | | ND | ND | ND | ND | ND | ND |
| 28 | | ND | ND | ND | ND | ND | ND |

**TABLE XI(A)**

| Concentration of (S)-Methoprene in Dog Blood Following Oral Administration at 50 mg/kg | | | | | | | |
|---|---|---|---|---|---|---|---|
| No. of Days | Dog No.: | (S)-Methoprene Concentration (ppb) | | | | | |
| | | #529 | #528 | #98 | #534 | #535 | #60 |
| 0 | | ND | ND | ND | ND | ND | ND |
| 1 | | 172 | 165 | 97.2 | 146 | 239 | 117 |
| 3 | | 52.1 | 63.6 | ND | 94.2 | 93.3 | 36.0 |
| 5 | | ND | ND | ND | 66.8 | 47.7 | ND |
| 7 | | 55.7 | ND | ND | ND | ND | ND |
| 10 | | ND | ND | ND | ND | ND | ND |
| 14 | | ND | ND | ND | ND | ND | ND |
| 17 | | ND | ND | ND | ND | ND | ND |
| 21 | | ND | ND | ND | ND | ND | ND |
| 28 | | ND | ND | ND | ND | ND | ND |

**TABLE XIB)**

| Concentration of (S)-Methoprene in Dog Blood of Untreated Dogs | | | | | | | |
|---|---|---|---|---|---|---|---|
| No. of Days | Dog No.: | (S)-Methoprene Concentration (ppb) | | | | | |
| | | #380 | #527 | #531 | #534 | #536 | #538 |
| 0 | | ND | ND | ND | ND | ND | ND |
| 1 | | ND | ND | ND | ND | ND | ND |
| 3 | | ND | ND | ND | ND | ND | ND |
| 5 | | ND | ND | ND | ND | ND | ND |
| 7 | | ND | ND | ND | ND | ND | ND |
| 10 | | ND | ND | ND | ND | ND | ND |
| 14 | | ND | ND | ND | ND | ND | ND |
| 17 | | ND | ND | ND | ND | ND | ND |
| 21 | | ND | ND | ND | ND | ND | ND |
| 28 | | ND | ND | ND | ND | ND | ND |

The data in these tables clearly indicate that the greater proportion of the ovicide resides in the hair, and remains present in the hair long after the ovicide is no longer detectable in the blood.

The foregoing is offered primarily for purposes of illustration. It will be readily apparent to those skilled in the art that the proportions, dosages, methods of administration, formulations, and other parameters of the methods described herein may be further modified or substituted in various ways.

## Claims

1. Use of a 2,4-dienoic acid, salt or ester insecticide for the manufacture of a pesticide for systemic administration to a terrestrial mammal for controlling fleas in the coat of said mammal, **characterized in that** the amount of insecticide that is at least about 80% lethal to developing fleas that are in contact with the skin or hair of said mammal yet below a concentration in the blood of said mammal that is about 50% lethal to developing fleas that arise from adult fleas which feed directly on said blood is equivalent to a daily dosage of from 0.3 to 15.0 mg/kg of body weight of said mammal per day.

2. Use according to claim 1 **characterized in that** the amount of insecticide administered is equivalent to a daily dosage of from 1.0 to 5.0 mg/kg of body weight of said mammal per day.

3. Use according to claim 1 **characterized in that** the amount of insecticide administered is less than that needed to maintain a concentration of 70 parts per billion, by weight thereof in the blood of said mammal.

4. Use according to claim 1 **characterized in that** the insecticide is administered orally.

5. Use according to claim 1 **characterized in that** said terrestrial mammal is a dog or cat.

6. Use according to claim 1 **characterized in that** said terrestrial mammal is a dog.

7. Use according to claim 1 **characterized in that** said insecticide is a compound having the formula 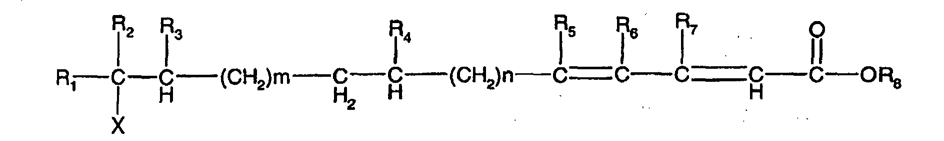 in which:
R₁ is C₁-C₆ alkyl;
R₂ is H, methyl or ethyl;
R₃ is H or methyl;
R₄ is methyl or ethyl;
R₅ is H or methyl;
R₆ is H or methyl;
R₇ is methyl or ethyl;
R₈ is a member selected from the group consisting of H, C₁-C₆ alkyl, C₃-C₆ alkenyl, C₃-C₆ alkynyl, C₃-C₈ cycloalkyl, phenyl, naphthyl, C₇-C₁₂ aralkyl, and cations of metals selected from the group consisting of lithium, sodium, potassium, calcium, strontium, copper manganese, and zinc;
X is a member selected from the group consisting of Br, Cl, Fl and OR₉, in which R₉ is a member selected from the group consisting of H, C₁-C₆ alkyl, and C₁-C₆ alkanoyl;
m is zero, 1, 2, or 3; and
n is zero, 1, 2, or 3.

8. Use according to claim 7 **characterized in that**
R₁ is methyl or ethyl;
R₂ is methyl or ethyl;
R₇ is methyl;
R₈ is a member selected from the group consisting of C₁-C₆ alkyl and C₃-C₆ alkynyl;
X is chloro or OR₉; in which R₉ is a member selected from the group consisting of H, C₁-C₆ alkyl, and C₁-C₆ alkanoyl;
m is zero or 1; and
n is 1.

9. Use according to claim 7 **characterized in that**
R₁ is methyl or ethyl;
R₂ is methyl;
R₃ is H;
R₄ is methyl;
R₅ is H;
R₆ is H;
R₇ is methyl;
R₈ is a member selected from the group consisting of C₁-C₄ alkyl and C₃-C₄ alkynyl;
X is chloro or OR₉; in which R₉ is a member selected from the group consisting of H, C₁-C₆ alkyl, and C₁-C₆ alkanoyl;
m is 1; and
n is 1.

10. Use according to claim 7 **characterized in that**
R₁ is methyl or ethyl;
R₂ is methyl;
R₃ is H;
R₄ is methyl;
R₅ is H;
R₆ is H;
R₇ is methyl;
R₈ is a member selected from the group consisting of C₁-C₄ alkyl and C₃-C₄ alkynyl;
X is chloro or OR₉, in which R₉ is a member selected from the group consisting of H, methyl, ethyl, isopropyl, t-butyl, and acetyl;
m is 1; and
n is 1.

11. Use according to claim 7 **characterized in that**
R₁ is methyl or ethyl;
R₂ is methyl;
R₃ is H;
R₄ is methyl;
R₅ is H;
R₆ is H;
R₇ is methyl;
R₈ is a member selected from the group consisting of C₁-C₄ alkyl and C₃-C₄ alkynyl;
X is OR₉ in which R₉ is a member selected from the group consisting of methyl, ethyl, isopropyl, and *t*-butyl;
m is 1; and
n is 1.

12. Use according to claim 7 **characterized in that** said insecticide has a configuration selected from the group consisting of *E,E* and *Z,E*.

13. Use according to claim 7 **characterized in that** said insecticide has a an *E,E* configuration.

14. Use according to claim 7 **characterized in that** said insecticide is isopropyl 11 -methoxy-3,7,1 1-trimethyldodeca-2,4-dienoate.

15. Use according to claim 7 **characterized in that** said insecticide is isopropyl (*E,E*)-(7S)-1 1 -methoxy-3,7, 11 -trimethyldodeca-2,4-dienoate.

## Patentansprüche

1. Verwendung eines Diencarbonsäureinsektizids oder eines Salzes oder Esters hiervon zur Herstellung eines Pestizids für die systemische Verabreichung an einen Landsäuger zur Bekämpfung von Flöhen im Fell des Säugers, **dadurch gekennzeichnet, dass** die Menge des Insektizids, die mindestens ungefähr 80% lethal für sich entwickelnde Flöhe ist, die in Kontakt mit der Haut oder dem Haar des Säugers stehen, und doch unterhalb einer Konzentration im Blut des Säugers ist, die ungefähr 50% lethal für sich entwickelnde Flöhe ist, die aus erwachsenen Flöhen entstehen, die sich direkt vom Blut ernähren, einer täglichen Dosierung von 0,3 bis 15,0 mg/kg des Körpergewichts des Säugers pro Tag äquivalent ist.

2. Verwendung nach Anspruch 1, **dadurch gekennzeichnet, dass** die Menge des verabreichten Insektizids einer täglichen Dosierung von 1,0 bis 5,0 mg/kg des Körpergewichts des Säugers pro Tag äquivalent ist.

3. Verwendung nach Anspruch 1, **dadurch gekennzeichnet, dass** die Menge des verabreichten Insektizids geringer ist als jene, die benötigt wird, um eine Konzentration von 70 Teilen pro Billion des Gewichts im Blut des Säugers aufrechtzuerhalten.

4. Verwendung nach Anspruch 1, **dadurch gekennzeichnet, dass** das Insektizid oral verabreicht wird.

5. Verwendung nach Anspruch 1, **dadurch gekennzeichnet, dass** der Landsäuger ein Hund oder eine Katze ist.

6. Verwendung nach Anspruch 1, **dadurch gekennzeichnet, dass** der Landsäuger ein Hund ist.

7. Verwendung nach Anspruch 1, **dadurch gekennzeichnet, dass** das Insektizid eine Verbindung ist, mit der Formel 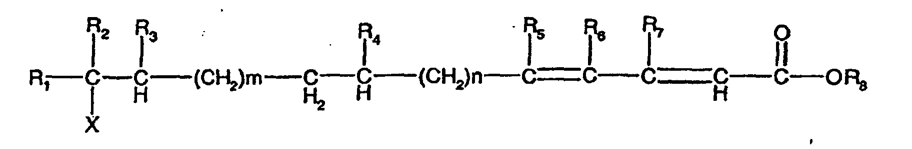 in der:
R₁ für C₁-C₆ Alkyl steht,
R₂ für H, Methyl oder Ethyl steht,
R₃ für H oder Methyl steht,
R₄ für Methyl oder Ethyl steht,
R₅ für H oder Methyl steht,
R₆ für H oder Methyl steht,
R₇ für Methyl oder Ethyl steht,
R₈ ein Rest ausgewählt aus der Gruppe ist, die besteht aus H, C₁-C₆ Alkyl, C₃-C₆ Alkenyl, C₃-C₆ Alkinyl C₃-C₈ Cycloalkyl, Phenyl, Naphthyl, C₇-C₁₂ Aralkyl und Kationen von Metallen besteht, die aus der Gruppe ausgewählt sind, die besteht aus Lithium, Natrium, Kalium, Calcium, Strontium, Kupfer, Mangan und Zink,
X ein Rest ausgewählt aus der Gruppe ist, die aus Br, Cl, F und OR₉ besteht, in der R₉ ein Rest ausgewählt aus der Gruppe ist, die aus H, C₁-C₆ Alkyl und C₁-C₆ Alkanoyl besteht,
m für 0, 1, 2 oder 3 steht und
n für 0, 1, 2 oder 3 steht.

8. Verwendung nach Anspruch 7, **dadurch gekennzeichnet, dass**
R₁ für Methyl oder Ethyl steht,
R₂ für Methyl oder Ethyl steht,
R₇ für Methyl steht,
R₈ ein Rest ausgewählt aus der Gruppe ist, die aus C₁-C₆ Alkyl und C₃-C₆ Alkinyl besteht,
X für Chlor oder OR₉ steht, wobei R₉ ein Rest ausgewählt aus der Gruppe ist, die aus H, C₁-C₆ Alkyl und C₁-C₆ Alkanoyl besteht,
m für 0 oder 1 steht und
n für 1 steht.

9. Verwendung nach Anspruch 7, **dadurch gekennzeichnet, dass**
R₁ für Methyl oder Ethyl steht,
R₂ für Methyl steht,
R₃ für H steht,
R₄ für Methyl steht,
R₅ für H steht,
R₆ für H steht,
R₇ für Methyl steht,
R₈ ein Rest ausgewählt aus der Gruppe ist, die aus C₁-C₄ Alkyl und C₃-C₄ Alkinyl besteht,
X für Chlor oder OR₉ steht, wobei R₉ ein Rest ausgewählt aus der Gruppe ist, die aus H, C₁-C₆ Alkyl und
C₁-C₆ Alkanoyl besteht,
m für 1 steht und
n für 1 steht.

10. Verwendung nach Anspruch 7, **dadurch gekennzeichnet, dass**
R₁ für Methyl oder Ethyl steht,
R₂ für Methyl steht,
R₃ für H steht,
R₄ für Methyl steht,
R₅ für H steht,
R₆ für H steht,
R₇ für Methyl steht,
R₈ ein Rest ausgewählt aus der Gruppe ist, die aus C₁-C₄ Alkyl und C₃-C₄ Alkinyl besteht,
X für Chlor oder OR₉ steht, wobei R₉ ein Rest ausgewählt aus der Gruppe ist, die aus H, Methyl, Ethyl, Isopropyl, t-Butyl und Acetyl besteht,
m für 1 steht und
n für 1 steht.

11. Verwendung nach Anspruch 7, **dadurch gekennzeichnet, dass**
R₁ für Methyl oder Ethyl steht,
R₂ für Methyl steht,
R₃ für H steht,
R₄ für Methyl steht,
R₅ für H steht,
R₆ für H steht,
R₇ für Methyl steht,
R₈ ein Rest ausgewählt aus der Gruppe ist, die aus C₁-C₄ Alkyl und C₃-C₄ Alkinyl besteht,
X für OR₉ steht, wobei R₉ ein Rest ausgewählt aus der Gruppe ist, die aus Methyl, Ethyl, Isopropyl und t-Butyl besteht,
m für 1 steht und
n für 1 steht.

12. Verwendung nach Anspruch 7, **dadurch gekennzeichnet, dass** das Insektizid eine Konfiguration hat, die aus der Gruppe ausgewählt ist, die aus E,E und Z,E besteht.

13. Verwendung nach Anspruch 7, **dadurch gekennzeichnet, dass** das Insektizid eine E,E-Konfiguration hat.

14. Verwendung nach Anspruch 7, **dadurch gekennzeichnet, dass** das Insektizid Isopropyl-11-methoxy-3,7,11-trimethyldodeca-2,4-dienoat ist.

15. Verwendung nach Anspruch 7, **dadurch gekennzeichnet, dass** das Insektizid Isopropyl-(E,E)-(7S)-11-methoxy-3,7,1 1-trimethyldodeca-2,4-dienoat ist.

## Revendications

1. Utilisation d'un insecticide d'acide 2,4-diénoïque, d'un de ses sels ou esters, pour la fabrication d'un pesticide pour l'administration systémique à un mammifère terrestre pour combattre les puces dans le pelage dudit mammifère, **caractérisée en ce que** la quantité d'insecticide, qui est léthale à au moins environ 80 % pour les puces en développement qui sont en contact avec la peau ou le pelage dudit mammifère, encore inférieure à une concentration dans le sang dudit mammifère qui est léthale à environ 50 % pour les puces en développement issues de puces adultes qui se nourrissent directement dudit sang, est équivalente à une posologie journalière de 0,3 à 15,0 mg/kg de poids corporel dudit mammifère par jour.

2. Utilisation suivant la revendication 1, **caractérisée en ce que** la quantité d'insecticide administrée est équivalente à une posologie journalière de 1,0 à 5,0 mg/kg de poids corporel dudit mammifère par jour.

3. Utilisation suivant la revendication 1, **caractérisée en ce que** la quantité d'insecticide administrée est inférieure à celle qui est nécessaire pour maintenir une concentration de 70 parties par milliard, en poids de celle-ci, dans le sang dudit mammifère.

4. Utilisation suivant la revendication 1, **caractérisée en ce que** l'insecticide est administré par voie orale.

5. Utilisation suivant la revendication 1, **caractérisée en ce que** ledit mammifère terrestre est un chien ou un chat.

6. Utilisation suivant la revendication 1, **caractérisée en ce que** ledit mammifère terrestre est un chien.

7. Utilisation suivant la revendication 1, **caractérisée en ce que** ledit insecticide est un composé répondant à la formule : 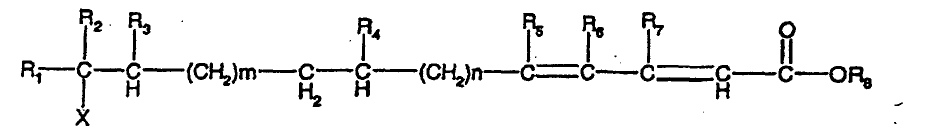 dans laquelle :
R₁ est un groupe alkyle en C₁ à C₆;
R₂ représente H, un groupe méthyle ou éthyle ;
R₃ représente H ou un groupe méthyle ;
R₄ est un groupe méthyle ou éthyle ;
R₅ représente H ou un groupe méthyle ;
R₆ représente H ou un groupe méthyle ;
R₇ est un groupe méthyle ou éthyle ;
R₈ est choisi dans le groupe constitué par H, un groupe alkyle en C₁ à C₆, alcényle en C₃ à C₆, alcynyle en C₃ à C₆, cycloalkyle en C₃ à C₈, phényle, naphtyle, aralkyle en C₇ à C₁₂, et des cations de métaux choisis dans le groupe constitué par le lithium, le sodium, le potassium, le calcium, le strontium, le cuivre-manganèse et le zinc ;
X est choisi dans le groupe constitué par Br, Cl, Fl et OR₉, où R₉ est choisi dans le groupe constitué par H, un groupe alkyle en C₁ à C₆ et alcanoyle en C₁ à C₆ ;
m vaut zéro, 1, 2 ou 3 ; et
n vaut zéro, 1, 2 ou 3.

8. Utilisation suivant la revendication 7, **caractérisée en ce que** :
R₁ est un groupe méthyle ou éthyle ;
R₂ est un groupe méthyle ou éthyle ;
R₇ est un groupe méthyle ;
R₈ est choisi dans le groupe constitué par un groupe alkyle en C₁ à C₆ et alcynyle en C₃ à C₆ ;
X est un groupe chloro ou OR₉ ; où R₉ est choisi dans le groupe constitué par H, un groupe alkyle en C₁ à C₆ et alcanoyle en C₁ à C₆ ;
m vaut zéro ou 1 ; et
n vaut 1.

9. Utilisation suivant la revendication 7, **caractérisée en ce que** :
R₁ est un groupe méthyle ou éthyle ;
R₂ est un groupe méthyle ;
R₃ représente H ;
R₄ est un groupe méthyle ;
R₅ représente H ;
R₆ représente H ;
R₇ est un groupe méthyle ;
R₈ est choisi dans le groupe constitué par un groupe alkyle en C₁ à C₄ et alcynyle en C₃ ou C₄ ;
X est un groupe chloro ou OR₉ ; où R₉ est choisi dans le groupe constitué par H, un groupe alkyle en C₁ à C₆ et alcanoyle en C₁ à C₆ ;
m vaut 1 ; et
n vaut 1.

10. Utilisation suivant la revendication 7, **caractérisée en ce que** :
R₁ est un groupe méthyle ou éthyle ;
R₂ est un groupe méthyle ;
R₃ représente H ;
R₄ est un groupe méthyle ;
R₅ représente H ;
R₆ représente H ;
R₇ est un groupe méthyle ;
R₈ est choisi dans le groupe constitué par un groupe alkyle en C₁ à C₄ ou alcynyle en C₃-C₄ ;
X est un groupe chloro ou OR₉, où R₉ est choisi dans le groupe constitué par H, un groupe méthyle, éthyle, isopropyle, t-butyle et acétyle ;
m vaut 1 ; et
n vaut 1.

11. Utilisation suivant la revendication 7, **caractérisée en ce que** :
R₁ est un groupe méthyle ou éthyle ;
R₂ est un groupe méthyle ;
R₃ représente H ;
R₄ est un groupe méthyle ;
R₅ représente H ;
R₆ représente H ;
R₇ est un groupe méthyle ;
R₈ est choisi dans le groupe constitué par un groupe alkyle en C₁ à C₄ et alcynyle en C₃-C₄ ;
X est un groupe OR₉ dans lequel R₉ est choisi dans le groupe constitué par les groupes méthyle, éthyle, isopropyle et t-butyle ;
m vaut 1 ; et
n vaut 1.

12. Utilisation suivant la revendication 7, **caractérisée en ce que** ledit insecticide a une configuration choisie dans le groupe consistant en les configurations *E,E* et *Z,E*.

13. Utilisation suivant la revendication 7, **caractérisée en ce que** ledit insecticide a une configuration *E,E*.

14. Utilisation suivant la revendication 7, **caractérisée en ce que** ledit insecticide est le 11-méthoxy-3,7,11-triméthyldodéca-2,4-diénoate d'isopropyle.

15. Utilisation suivant la revendication 7, **caractérisée en ce que** ledit insecticide est le (*E,E*)-(7S)-11-méthoxy-3,7,11-triméthyldodéca-2,4-diénoate d'isopropyle.
